# EUROPEAN PATENT SPECIFICATION

(11) **EP 4 382 070 B1**
(45) Date of publication and mention of the grant of the patent: **16.07.2025**
(21) Application number: 22306833.9
(22) Date of filing: 09.12.2022
(51) Int. Cl.: A61B 34/30, A61B 34/00, B25J 7/00, A61B 17/00

(54) **RESONATING SYSTEM - LIMP**
RESONANZSYSTEM - LIMP
SYSTÈME RÉSONANT - LIMP

(43) Date of publication of application: 12.06.2024
(73) Proprietor: Robeaute, 75014 Paris (FR)
(72) Inventor: DUPLAT, Bertrand, 75002 Paris (FR); FRANCOIS, Quentin, 75002 Paris (FR); OULMAS, Ali, 75002 Paris (FR)
(74) Representative: Icosa

(56) References cited:
- US-A1- 2010 264 776
- US-A1- 2020 305 796
- US-A1- 2022 273 383
- YANG LIDONG ET AL: "A Miniature Flexible-Link Magnetic Swimming Robot With Two Vibration Modes: Design, Modeling and Characterization", IEEE ROBOTICS AND AUTOMATION LETTERS, IEEE, vol. 2, no. 4, 1 October 2017 (2017-10-01), pages 2024 - 2031, XP011655623, DOI: 10.1109/LRA.2017.2718104
- QINXUE PAN ET AL: "Paddling type of microrobot in pipe", 2009 IEEE INTERNATIONAL CONFERENCE ON ROBOTICS AND AUTOMATION : (ICRA) ; KOBE, JAPAN, 12 - 17 MAY 2009, IEEE, PISCATAWAY, NJ, USA, 12 May 2009 (2009-05-12), pages 2995 - 3000, XP031483242, ISBN: 978-1-4244-2788-8
- DEASUNG JANG ET AL: "Targeted drug delivery technology using untethered microrobots: a review", JOURNAL OF MICROMECHANICS AND MICROENGINEERING, INSTITUTE OF PHYSICS PUBLISHING, BRISTOL, GB, vol. 29, no. 5, 20 March 2019 (2019-03-20), pages 53002, XP020336337, ISSN: 0960-1317, [retrieved on 20190320], DOI: 10.1088/1361-6439/AB087D
- MOHAND-OUSAID ABDENBI ET AL: "Compact Digital Microrobot Based on Multistable Modules", IEEE ROBOTICS AND AUTOMATION LETTERS, IEEE, vol. 6, no. 2, 22 February 2021 (2021-02-22), pages 1926 - 1933, XP011841082, DOI: 10.1109/LRA.2021.3061003
- QIAO CHEN ET AL: "Microfabricated Bistable Module for Digital Microrobotics", vol. 6, no. 1-2, 21 January 2011 (2011-01-21), pages 1 - 12, XP002660690, ISSN: 1865-3928, Retrieved from the Internet <URL:http://hal.archives-ouvertes.fr/docs/00/55/84/15/PDF/JMNM2010_Yassine.pdf> [retrieved on 20111006], DOI: 10.1007/S12213-010-0025-2

## Description

### FIELD OF INVENTION

The present invention relates to a steering system of a micro-robot aimed at circulating through a viscous environment, in particular inside a human body.

### BACKGROUND OF INVENTION

The ability to reach deep and functional structures without damage is a major challenge in mini-invasive surgery, especially in neurosurgery. Thanks to microtechnologies, it becomes possible to send a fully autonomous microrobot inside an organ of a subject, such as a brain. However, the propulsion of a microrobot in an environment at low Reynolds number, as in the brain, is a challenge because of absence of inertia and presence of relatively high drag forces due to the small size of the microrobot. Another important requirement is that the microrobot should be capable of moving in an organ while limiting as much as possible the physiological damage that its passage causes to the organ.

US 2022/273383 A1 discloses a micro-robot aimed at circulating through a viscous environment, in particular inside a human body.

In this context, the invention is intended to propose a microrobot having a highly efficient propulsion mechanism in a viscous environment at low Reynolds number, while preserving as much as possible the integrity of the environment in which it is displaced.

### SUMMARY

The invention is defined in appended independent claim 1. Further embodiments are defined in appended dependent claims.

This invention thus relates to a micro-robot configured to move along a propulsion direction by vibrations, said micro-robot comprising a body configured to vibrate, and an actuator configured to generate vibrations causing the micro-robot to move, said micro-robot further comprising a steering system which comprises a resonating structure configured to be secured to the micro-robot, the resonating structure comprising:
- a steering structure aimed at controlling the propulsion direction,
- a distribution of weight-resonators, each weight-resonator being configured to be activated by a proper activation resonance frequency, the respective proper activation resonance frequencies of the weight-resonators being different from each other,
**wherein** the actuator is configured to generate vibrations in a range of frequencies including the proper activation resonance frequency of each weight-resonator, **wherein** the resonating structure displays at least two states:
- at least one activated steering state, in which at least one of the weight-resonators is activated at the proper activation resonance frequency to change the propulsion direction of the micro-robot,
- a non-activated steering state, in which none of the weight-resonators is activated at its proper activation resonance frequency so as to maintain the propulsion direction of the micro-robot.

Thus, this solution achieves the above objective. In particular, it allows the obtaining of a rotation of the micro-robot solely based on the movements (energy) generated by the micro-robot itself (more precisely by the micro-motor of the micro-robot), thus avoiding the addition of extra-energy which might lead to further elements or devices to be added inside the patient or additional energy to be conveyed to the micro-robot, all which might lead to increase risks of damaging the environment in which the micro-robot moves.

The device according to the invention may include one or more of the following characteristics, taken in isolation from one another or in combination with one another:
- the resonating structure may display several activated steering states, each activated steering state being associated to a different proper activation resonance frequency,
   o the at least one activated steering state, is a state in which the configuration and movement of the resonating structure aim at changing the propulsion direction of the micro-robot,
   o the non-activated steering state, is a state in which the configuration and movement of the resonating structure aim at maintaining the propulsion direction of the micro-robot,
- the resonating structure may further comprise a distribution of multi-stable elements, each multi-stable element being deformable between at least two stable configurations:
   - at least a first stable configuration when the resonating structure is in its activated steering state,
   - at least a second stable configuration when the resonating structure is in its non-activated steering state,
- the multi-stable element may be a bi-stable pre-compressed beam displaying the first and the second stable configurations, the pre-compressed beam being bent in a first direction towards the body of the micro-robot in the first stable configuration and being bent in a second direction away from the body of the micro-robot in the second stable configuration,
- the steering structure may be a retractable steering foil displaying an open and a retracted configuration with regards to the body of the micro-robot, the steering foil configuration being determined by the steering state of the resonating structure:
   - the non-activated steering state of the resonating structure induces the retracted configuration of the retractable steering foil, and
   - the activated steering state of the resonating structure induces the open configuration of the retractable steering foil,
- each multi-stable element may be a multi-stable shell comprising a stack of several sheets,
- the steering system may further comprise at least one mobile cilium, the at least one cilium being configured to be put in motion by the vibration of the body of the micro-robot,
- the resonating structure may comprise at least one strand of a propulsion spring comprised within the body,
- the activation of the resonating structure in its at least one activated steering state may induce the retractation of the at least one strand of the propulsion spring,
- the body of the micro-robot may present a global circular symmetry along a propulsion axis parallel to the propulsion direction, wherein the resonating structure is further part of said body, and wherein the activation of the resonating structure into one of its activated steering states induces a break in the global circular symmetry of the body,
- the at least one cilium may be part of the resonating structure, the at least one cilium presenting a first movement intensity in the non-activated steering state and a second movement intensity in the activated steering state, the second movement intensity being different than the first movement intensity,
- each cilium may comprise a weight-resonator.

### BRIEF DESCRIPTION OF THE DRAWINGS

The invention will be better understood, and other aims, details, characteristics and advantages thereof will emerge more clearly on reading the detailed explanatory description which follows, of embodiments of the invention given by way of illustration. purely illustrative and non-limiting examples, with reference to the accompanying drawings.
- **figure 1a** is a general schematic side view of the micro robot comprising a steering system according to a first embodiment of the present invention,
- **figure 1b** is a general schematic side view of the micro robot comprising a steering system according to a second embodiment of the present invention,
- **figure 2a** is a detailed schematic side view of the steering system according to the first embodiment of the present invention,
- **figure 2b** is a detailed schematic side view of the steering system according to the second embodiment of the present invention,
- **figure 3a** is a detailed schematic view from above of the steering system according to the first embodiment of the present invention,
- **figure 3b** is a detailed schematic view from above of the steering system according to the second embodiment of the present invention,
- **figure 4** is a step-by-step schematic view of the activation of the steering system according to the first embodiment,
- **figure 5a** is a general schematic side view of the micro robot comprising a steering system according to a third embodiment of the present invention,
- **figure 5b** is a detailed schematic side view of the steering system according to the third embodiment of the present invention,
- **figure 5c** is a detailed schematic view from above of the steering system according to the third embodiment of the present invention,
- **figure 6** is a step-by-step schematic view of the activation of the steering system according to the third embodiment of the present invention,
- **figure 7a** is a general schematic side view of the micro robot comprising a steering system according to a fourth embodiment of the present invention,
- **figure 7b** is a detailed schematic side view of the steering system according to the fourth embodiment of the present invention,
- **figure 7c** is a detailed schematic view from above of the steering system according to the fourth embodiment of the present invention,
- **figure 8** is a step-by-step schematic view of the activation of the steering system according to the fourth embodiment of the present invention.

### DETAILED DESCRIPTION

Please note that in the present application, the term "weight-resonator" is given a wide definition: It is not necessarily just resonators based on their weight. They might also include foils and bi-stable or multi-stable elements which enable to play with the shape of the part and the internal tensions that generate several stable states. Those enable to go from one stable state to another by bringing energy. This is achieved, as will be explained further below in detail, by the change of a resonance frequency which maximizes the available/conducted energy.

### Embodiments

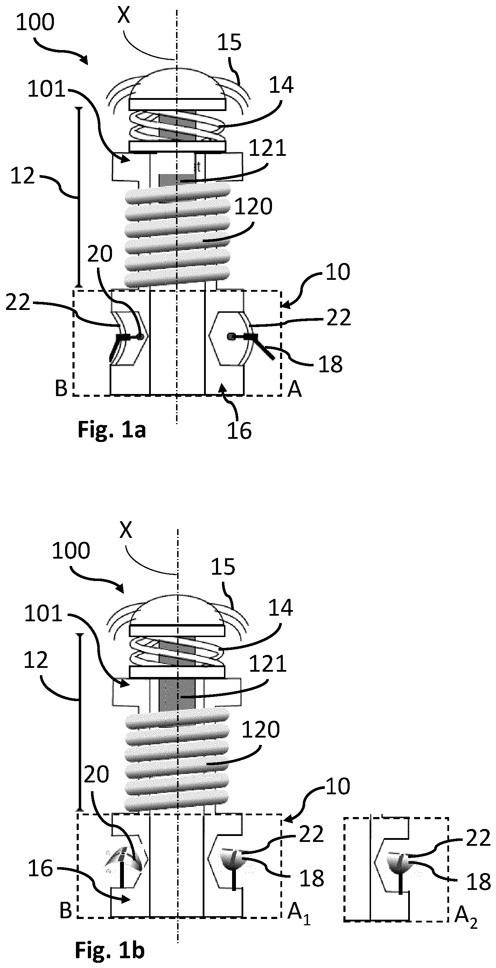

As can be seen on figures 1a and 1b, the steering system 10 according to the present invention, is aimed at being part of a micro-robot 100 configured to move inside a fluidic environment, more precisely along a propulsion direction following a propulsion axis X. This movement along the propulsion direction happens by vibration inside the fluidic environment.

The micro-robot 100 thus comprises a body 101 comprising an actuator 12 configured to generate vibrations. More precisely, in the represented embodiments, the actuator 12 is a vibrating motor 12 which comprises a coil 120 and a magnet 121. The coil 120 extends along the propulsion direction X and surrounds the magnet 121. The magnet 121 is activable by the coil 120 and is configured to move back and forth along the propulsion direction X. The movement of the magnet 121 induces a compression/decompression movement of a propulsion spring 14 also part of the vibrating motor 12 and also extending along the propulsion direction X. Said propulsion spring 14 allows the movement of a series of external pilis or cilia 15 which enable the micro-robot 100 to be put in motion inside the fluidic environment.

Many fluidic environments can be targeted, but specifically all bodily fluids are concerned. Those bodily fluid are, for example, blood, cerebra spinal fluids, urine, bile liquid, lymph fluid, aqueous humor. Those fluids all present a viscosity close to that of water.

As can be further seen on figures 1a and 1b, the steering system 10 according to the present invention comprises an elongated resonating structure 16 aimed at being secured to the micro-robot 100.

This elongated resonating structure 16 comprises:
- a steering structure 18 aimed at controlling the propulsion direction,
- a distribution of weight-resonators 20 configured to be activated over a proper (or given) activation resonance frequency f_{A}, f_{A1}, f_{A2} and deactivated under a proper (or given) deactivation frequency f_{B}.

The respective proper activation resonance frequencies f_{A}, f_{A1}, f_{A2} of each weight-resonator 20 is different from the proper activation resonance frequencies f_{A}, f_{A1}, f_{A2} of the other weight-resonators 20. The actuator 12 is configured to generate vibrations in a range of frequencies including the proper activation resonance frequency f_{A}, f_{A1}, f_{A2} of each weight-resonator 20.The elongated resonating structure 16 displays at least two states:
- at least one activated steering state A, A₁, A₂, in which the configuration and movement of the elongated resonating structure 16 are configured to change the propulsion direction of the micro-robot 100,
- a non-activated steering state B, in which the configuration and movement of the elongated resonating structure 16 are configured to maintain the propulsion direction of the micro-robot 100.

Regardless of the embodiment, the state B, A, A₁, A₂ of the elongated resonating structure 16, is determined by the activation or deactivation of the weight-resonators 20 as will be explained further below.

As will be explained further below, the activation and deactivation frequencies f_{B}, f_{A}, f_{A1}, f_{A2} of the weight-resonator 20 are induced by the vibrations of the micro-robot body 101 (mor particularly the vibrations induced by the micro-motor comprised within the body 101 of the micro-robot) when the micro-robot 100 is moving along its propulsion direction.

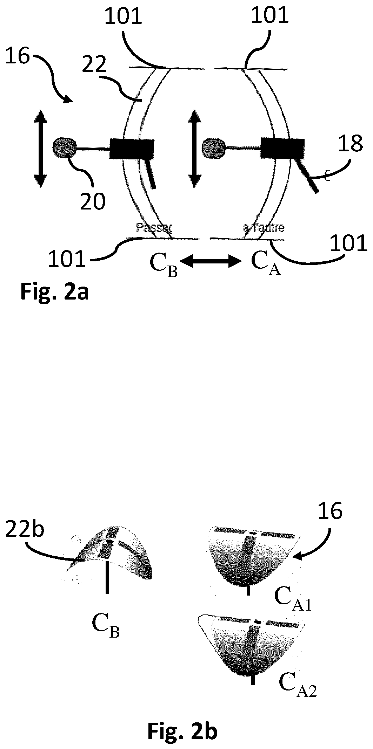

More particularly regarding figures 1a and 1b, the elongated resonating structure 16 further comprises a distribution of multi-stable element 22 secured to the micro-robot 100. Each multi-stable element 22 displays at least two stable configurations and is deformable between those at least two stable configurations:
- at least a first stable configuration C_{A}, C_{A1}, C_{A2} when the resonating structure 16 is in its activated steering state A, A₁, A₂ (see figures 2a, 2b),
- at least a second stable configuration C_{B} when the resonating structure 16 is in its non-activated steering state B (see figures 2a, 2b).

Depending on the embodiments, the multi-stable element 22, the weight-resonator 20 and the steering structure 18 can be the same technical element or distinct elements. For example, in the embodiment depicted on figure 1a, the multi-stable element 22, the weight resonator 20 and the steering structure 18 are all distinct technical elements. However, regarding the embodiment depicted on figure 1b, the multi-stable element 22, the weight-resonator 20 and the steering structure 18 are the same technical element. In this case, the different proper activation frequencies f_{A}, f_{A1}, f_{A2} could be dedicated to, for example, the control the shape of the weight-resonator 20 (or multi-stable element 22). One could have a frequency for semi-closure, another for full closure, for example.

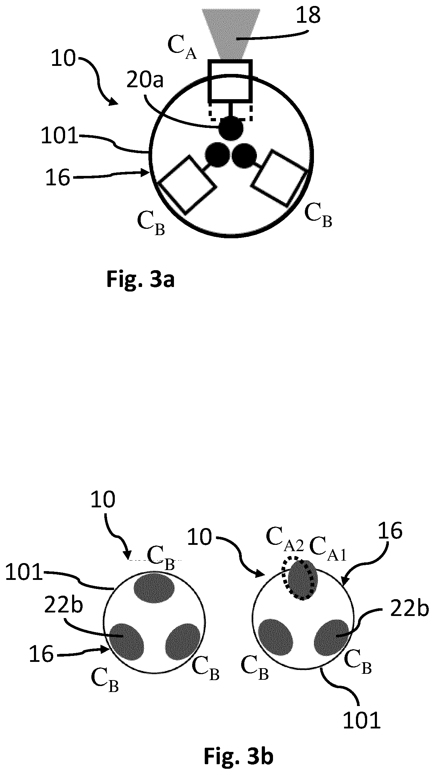

More precisely regarding the embodiment depicted on figures 1a, 2a and 3a, the elongated resonating structure 16 comprises three multi-stable elements 22, equidistantly distributed around the body 101 of the micro-robot 100 (see figure 3a). Each multi-stable elements 22 is associated to a weight-resonator 20 having a specific proper activation frequency f_{A}. This specific proper activation frequency f_{A} being different from the proper activation frequencies f_{A} of the other weight-resonator 20 associated to the other multi-stable elements 22. In this example, each multi-stable element 22 is a bi-stable pre-compressed beam 22 between 100µm and a few mm in length, made of polymers, glass or metal such as stainless steel or alliage. In this particular embodiment, each beam 22 connects the steering structure 18 and the weight-resonator 20. Each beam 22 has two extremities, and each extremity is secured to the micro-robot 100. More precisely, each beam 22 is secured in a small open cavity of the body 101 of the micro-robot 100. As already mentioned, each beam 22 presents a first stable configuration C_{A} in which it bends inwards the cavity of the micro-robot 100 and a second stable configuration C_{B} in which it bends outwards the cavity of the micro-robot 100. The first stable configuration C_{A} corresponds to the activated steering state A, the second stable configuration C_{B} corresponds to the non-activated steering state B. In its non-activated steering state B, the pre-compressed beam 22 is bent in a first direction, away from the body 101 of the micro-robot 100. In its activated steering state A, the pre-compressed beam 22 is bent in a second direction, towards the body 101 of the micro-robot 100 and thus different from the first direction.

Still considering the embodiment on figures 1a and 2a, the steering structure 18 comprises a retractable steering foil 18 displaying an open and a retracted configuration with regards to the body 101 of the micro-robot 100 (see figure 2a). As will be explained later, the configuration of the steering foil 18 is determined by the state of the elongated resonating structure 16 of the steering system 10. The foils could be made of many different flexible materials such as metals such as copper or alliage metals, glass or polymers among others. Size can vary from about 100µm to a few mm. More precisely, the non-activated steering state B of the elongated resonating structure 16 induces a retracted configuration of the retractable steering foil 18. On the other hand, the activated steering state A of the elongated resonating structure 16 induces an open configuration of the retractable steering foil 18.

Still considering figure 1a, each weight-resonator 20 comprises a resonating mass connected to the beam 22. This mass can be of any suitable shape, for example a bead or a cube. Depending on the embodiment the resonating mass could also be embedded in the beam 22 by extra thickness or an extension of the shape of the beam 22. The activation of the resonating mass of the weight-resonator 20 thus induces a configuration change in the beam 22. The configuration change of the beam 22a further induces a configuration change of the corresponding steering foil 18.

In the embodiment presented in figures 1b, 2b and 3b, the multi-stable element 22 is a multi-stable shell 22 comprising a stack of several sheets. The multi-stable elements could be made of many different flexible materials who have this multi-stable state capacity when shaped appropriately such as copper. Size can vary from about 100µm to a few mm.

In this embodiment, the multi-stable shell 22 displays a non-activated steering state B, in this state none of the shells oppose a resistance to the movement of the system by blocking the flow of the liquid, and several activated steering states A₁, A₂. Each activated steering state A₁, A₂ is associated to a different proper activation resonance frequency f₁, f₂. In those activated states, a shell is positioned in a way that oppose the flow, this creates an asymmetry and induces a rotation in the direction to which the shell is exposed.

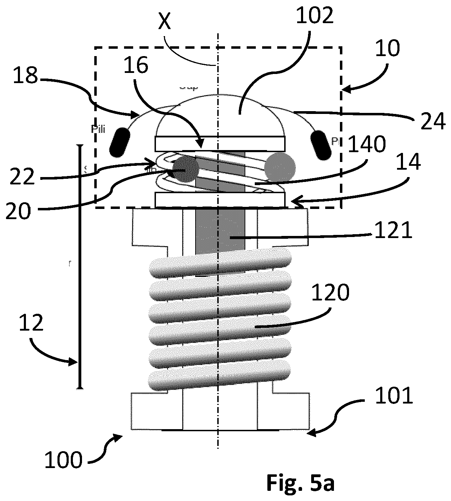

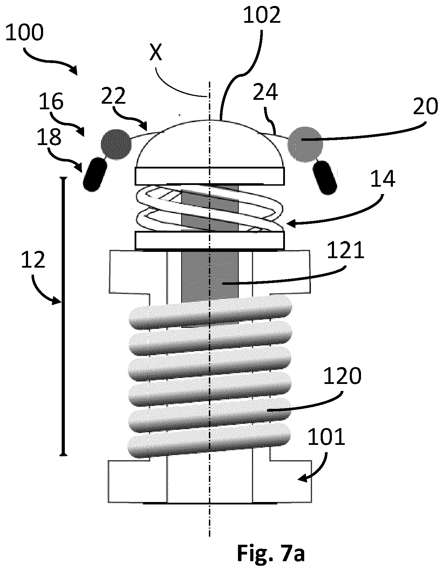

According to further embodiments depicted, in particular, on figures 5a and 7a, the steering system 10, and more particularly the elongated resonating structure 16, comprises at least one mobile cilium 24. In some embodiment, the elongated resonating structure 16 comprises at least one, preferably a group of mobile cilia 24. This at least one mobile cilium 24 can be part of the series of external pilis or cilia 15 which enable the micro-robot 100 to be put in motion inside the fluidic environment (see paragraph [009]). The at least one mobile cilium 24 may also be an independent technical element from said series of external pilis or cilia 15.

In the embodiments of figures 5a and 7a, the at least one cilium 24 is part of the steering structure 18. In this embodiment, the steering structure further is configured to enable a preferential activation of one single cilium 24 over the other cilia 24. In order to achieve this, the steering structure may comprise a foil carried by said at least one cilium 24. The foil can present different orientations depending on the configuration and/or position of the at least one cilium 24. Those different configurations/positions enable the steering structure 18 to control the propulsion direction of the micro-robot 100.

Each cilium 24 is secured to the body 101 of the micro-robot 100, preferably on the head portion of the micro-robot 100. More precisely, each 24 is secured to a mobile part 102 of the body 101 of the micro-robot 100. This mobile part 102 is connected to the propulsion spring 14 of the vibrating motor 12 and moves in accordance with said propulsion spring 14. Depending on the movements of the propulsion spring 14, said mobile part 102 can be centred or off-centred around the the propulsion direction X. Each cilium 24 is thus configured to be put in motion by the vibration of the micro-robot 100, more particularly by the movement of the propulsion spring 14 of the motor (actuator) 12.

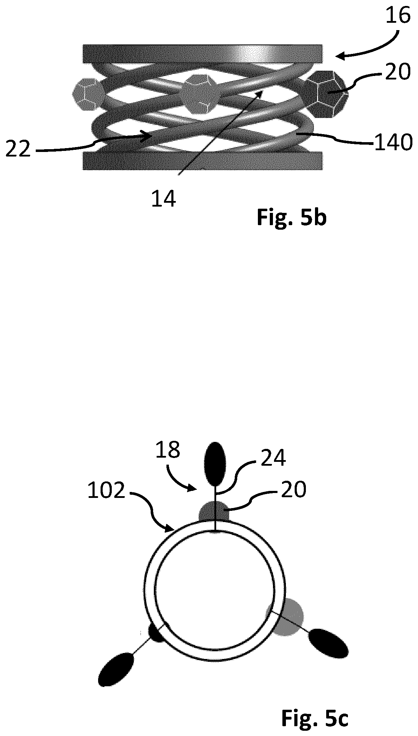

In this embodiment, the propulsion string 14 comprises several strands 140 from which some are equipped weight-resonators 20 (see figure 5b). Thus, in this embodiment, the elongated resonating structure 16 comprises at least one strand 140 of the propulsion spring 14 of the vibrating motor/actuator 12.

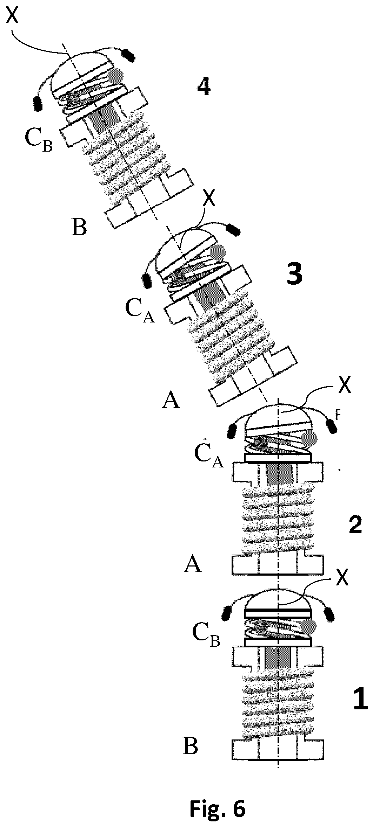

More particularly, in the embodiment depicted on figures 5a, 5b, 5c and 6, each strand 140 carrying a weight-resonator 20 is part of the multi-stable element 22 of the steering system 10. Each weight-resonator 20 is, similarly to the precedingly detailed embodiments, activable over a proper activation resonance frequency f_{A}, f_{A1}, f_{A2} and deactivated under a given deactivation frequency f_{B}. As can be seen on figure 5b, the propulsion spring 14 comprises three independent strands 140 and each strand 140 carries a weight-resonator 20 which is activable over a different proper activation frequency f_{A}, f_{A1}, f_{A2}. As hinted at in figure 5b, the different weight-resonator 20 present different sizes and shapes, leading to different proper activation frequencies f_{A}, f_{A1}, f_{A2}. In an alternative embodiment, the different proper activation frequencies f_{A}, f_{A1}, f_{A2} could be dedicated to, for example, the control the shape of the weight-resonator 20. One could have a frequency for semi-closure, another for full closure.

In this embodiment (figures 5a, 5b, 5c and 6), the activation of the elongated resonating structure 16 in its at least one activated steering state A, A₁, A₂ (see figure 6), and more particularly the activation of each weight-resonator 20 induces a retractation of the corresponding strand 140 of the propulsion spring 14.

More particularly, in this embodiment (figures 5a, 5b, 5c and 6), each strand 140 presents a first stable configuration C_{A} in which it presents a first length L_{A} corresponding to an activated steering state A₁, A₂ and a second stable configuration C_{B} in which it presents a second length L_{B}, corresponding to the non-activated steering state B. When the strand 140 is in its second stable configuration C_{B}, its length L_{B} is the same than the length of the other strands 140. In this second stable configuration, the mobile part 102 of the body 101 of the micro-robot 100, is centred with regards to an elongation axis X of the micro-robot 100. Said elongation axis X is the same as the propulsion axis X already mentioned previously. When the strand 140 is in its activated configuration C_{A}, its length changes and the mobile part 102 of the body 101 of the micro-robot 100 is off-centred. The activation of the elongated resonating structure 16 in one of its activated steering states A₁, A₂ thus induces a break in the global symmetry of the body 101 of the micro-robot 100.

Regarding the embodiments depicted respectively on figures 1a, 2a, 3a and 1b, 2b and 3b, it can also be said that the activation of the elongated resonating structure 16 in one of its activated steering states A₁, A₂ induces a break in the global symmetry of the body 101 of the micro-robot 100: the spreading of the foils 18a, 18b break the global rotational symmetry of the micro-robot 100.

The length of each strand 140 depends on the activation/inactivation of each associated weight -resonator 20. The length of each strand 140 thus depends on the vibration amplitude of the vibrating motor 12 and of the propulsion spring 14, going from about 0 (it does not move) to an amplitude in hundreds of µm.

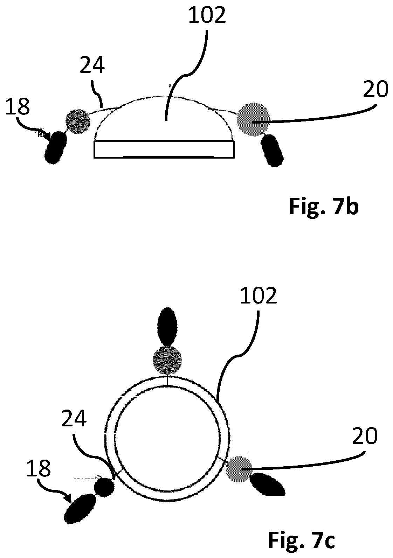

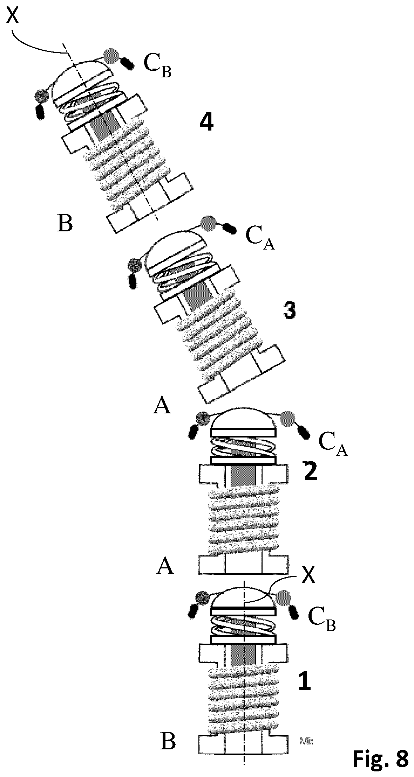

In the last depicted embodiment of the present invention (figures 7a, 7b, 7c and 8), the at least one cilium 24 is also part of the elongated resonating structure 16. However, it is also part of the steering structure 18 and of the multi-stable element 22. As in the previous embodiment, each cilium 24 carries a foil in order to enable some steering of the micro-robot 100.

In this embodiment, the weight-resonator 20 activable by the frequencies of the propulsion spring 14, are carried by the cilia 24. More particularly, each cilium 24 carries a weight-resonator 20 activable at a propre activation frequency f_{A}, f_{A1}, f_{A2}.

In this embodiment, the at least one cilium 24 presents at least two stable states C_{A}, C_{B}, each stable state C_{A}, C_{B} being associated to a movement intensity. This way, each cilium 24 presents a first stable state C_{B} correspondent to a first movement intensity I_{B}. This first stable state C_{B} corresponds to the non-activated state of the weight-resonator 20 carried by the cilium 24 and thus corresponds to the non-activated steering state B. The second stable state C_{A} corresponds to a second movement intensity I_{A} and thus to the activated steering state A. The second movement intensity I_{A} being higher than the first movement intensity I_{B}. All cilium 24 in the first stable state C_{B} are moving (back and forth movements) with the same first movement intensity I_{B}. Once one (or several) cilium (cilia) 24 is (are) activated, it (they) start(s) moving with a different intensity, the second movement intensity I_{A}, thus inducing a break in the general symmetry of the micro-robot 100 which leads to a disequilibrium and eventually to a direction change.

### Functioning

As mentioned above, for each embodiment, the activation of the weight-resonator 20 induces the elongated resonating structure 16 to enter its at least one activated steering state A, A₁, A₂. On the other hand, the deactivation of the weight-resonator 20 induces the elongated resonating structure 16 to enter its non-activated steering state B.

When the elongated resonating structure 16 is in its non-activated steering state B, the propulsion direction of the micro-robot 100 is maintained the same and the micro-robot 100 moves straight forward along the propulsion axis X. However, when the elongated resonating structure 16 enters its at least one activated steering state A, A₁, A₂, the micro-robot 100 rotates and changes its propulsion direction.

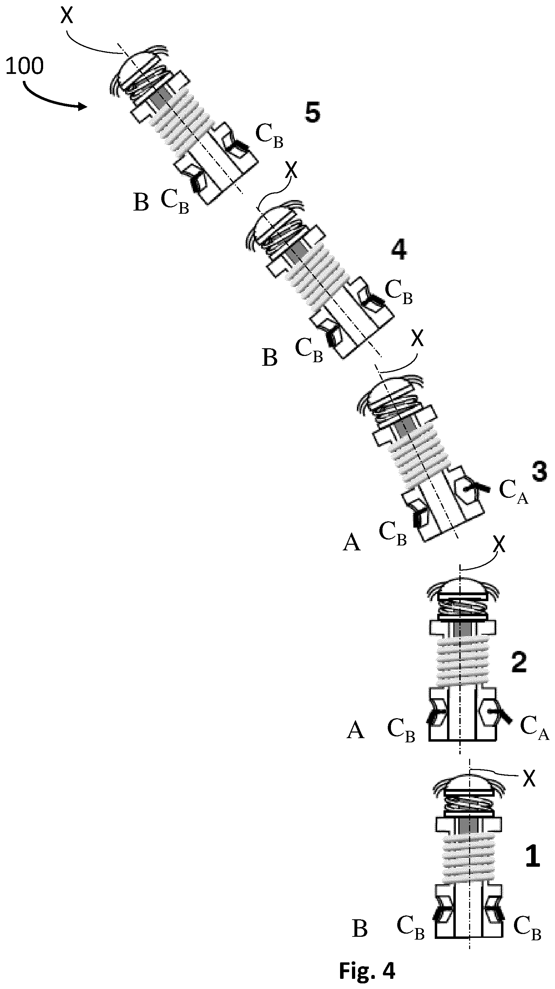

Considering the first embodiment (figures 1a, 2a, 3a, 4), the vibration of the propulsion spring 14 induces the micro-robot 100 to vibrate at given frequencies. When the micro-robot 100 vibrates at the proper activation resonance frequency f_{A} of one of the weight-resonators 20 of the elongated resonating structure 16, the considered weight-resonator 20 starts vibrating. This vibration induces a configuration change in its associated pre-compressed beam 22a from its inactivated configuration C_{B} to its activated configuration C_{A}. This configuration change leads the associated steering foil 18 to be expanded. The elongated resonating structure 16 finds itself in its steering activated state A. The expansion of the steering foil 18 induces a rotation of the micro-robot 100 and a redefinition of the propulsion direction X (see figure 6). When the vibration of the micro-robot 100 falls below the given deactivation frequency f_{B} of the weight-resonator 20, the weight-resonator 20 is deactivated and the pre-compressed beam 22 falls back into its inactivated configuration C_{B}. The steering foil 18 is thus retracted and the elongated resonating structure 16 falls back in its inactivated steering state B. The frequency and/or amplitude of the motor 12 is changed to go from one resonant frequency related to a specific state to another related to another state.

Regarding the second embodiment (figures 1b, 2b, 3b), the functioning is similar to the precedent embodiment, with exception that the vibrating of the propulsion spring 14 activates directly the multi-stable shell 22b, which changes configuration and induces the micro-robot 100 to rotate.

Considering the fourth embodiment (figures 5a, 5b, 5c, 6), it has to be specified that each cilium 24 vibrates at a given intensity, said vibration being induced by the vibrations of the vibrating motor (actuator) 12 of the micro-robot 100. When the micro-robot 100 vibrates at the proper activation resonance frequency f_{A} of one of the weight-resonators 20 of the elongated resonating structure 16, the considered weight-resonator 20 starts vibrating. This vibration induces a configuration change in its associated strand 140, more precisely a modification of its length from its second length L_{B} defining its inactivated state C_{B,} to its first length L_{A} defining its activated state C_{A}. This change in length induces a break in the global symmetry of the body 101 of the micro-robot 100 and the at least one cilium 24 undergoes a shift in its vibration axis, thus inducing a rotation of the micro-robot 100 (see figure 6).

Regarding the last embodiment (figures 7a, 7b, 7c and 8), when the micro-robot 100 vibrates at the proper activation resonance frequency f_{A} of one of the weight-resonators 20 of the elongated resonating structure 16, the considered weight-resonator 20 starts vibrating. This vibration induces a configuration change in the vibration intensity of the associated cilium 24. The cilium 24 then changes from its first movement intensity I_{B} (corresponding to the steering non-activated state B of the elongated resonating structure 16) to its movement intensity I_{A} (corresponding to the steering activated state A of the elongated resonating structure 16). The activated cilium 24 thus vibrates at a different speed and a different amplitude from the other cilia 24 (or the series of external pilis or cilia 15) and this induces a rotation of the micro-robot 100 (see figure 7).

This rotation of the micro-robot 100 most likely happen by a succession of jolts, creating a discrete cumulation of several small rotational movements leading to the desired final rotation. This enables an additional layer of precision and security.

The fact of being able to manage the 3D orientation of a device with only one linear actuator on which a user can change the activation frequency from a distance, gives a 2D plane of solutions (one axis = frequency, one axis = intensity) and one need solely to navigate in this plane to have the desired configuration, which is both more easy and more reliable.

## Claims

1. Micro-robot (100) configured to move along a propulsion direction by vibrations, said micro-robot (100) comprising a body (101) comprising an actuator (12) configured to generate vibrations causing the micro-robot (100) to move,
said micro-robot (100) further comprising a steering system (10) which comprises a resonating structure (16) configured to be secured to the micro-robot (100), the resonating structure (16) comprising:
- a steering structure (18) aimed at controlling the propulsion direction,
- a distribution of weight-resonators (20), each weight-resonator (20) being configured to be activated by a proper activation resonance frequency (f_{A}, f_{A1}, f_{A2}), the respective proper activation resonance frequencies (f_{A}, f_{A1}, f_{A2}) of the weight-resonators (20) being different from each other,
**wherein** the actuator (12) is configured to generate vibrations in a range of frequencies including the proper activation resonance frequency of each weight-resonator,
**wherein** the resonating structure (16) displays at least two states:
- at least one activated steering state (A, A₁, A₂), in which at least one of the weight-resonators is activated at the proper activation resonance frequency to change the propulsion direction of the micro-robot (100),
- a non-activated steering state (B), in which none of the weight-resonators is activated at its proper activation resonance frequency so as to maintain the propulsion direction of the micro-robot (100).

2. Micro-robot (100) according to the preceding claim, **wherein** the actuator 12 is a vibrating motor 12 which comprises a coil 120 extending along the propulsion direction X and surrounding a magnet 121, the magnet 121 being activable by the coil 120 and being configured to move back and forth along the propulsion direction X.

3. Micro-robot (100) according to any one of the preceding claims, **wherein** the resonating structure (16) displays several activated steering states (A₁, A₂), each activated steering state (A₁, A₂) being associated to a different proper activation resonance frequency (f_{A1}, f_{A2}).

4. Micro-robot (100) according to any one of the preceding claims, **wherein:**
- the at least one activated steering state (A, A₁, A₂), is a state in which the configuration and movement of the resonating structure (16) aim at changing the propulsion direction of the micro-robot (100),
- the non-activated steering state (B), is a state in which the configuration and movement of the resonating structure (16) aim at maintaining the propulsion direction of the micro-robot (100),

5. Micro-robot (100) according to any one of the preceding claims, **wherein** the resonating structure (16) further comprises a distribution of multi-stable elements (22), each multi-stable element (22) being deformable between at least two stable configurations:
- at least a first stable configuration (C_{A}) when the resonating structure (16) is in its activated steering state (A),
- at least a second stable configuration (C_{B}) when the resonating structure (16) is in its non-activated steering state (B).

6. Micro-robot (100) according to the preceding claim, **wherein** each multi-stable element (22) is a bi-stable pre-compressed beam (22) displaying the first and the second stable configurations (C_{A}, C_{B}), the pre-compressed beam (22) being bent in a first direction towards the body (101) of the micro-robot (100) in the first stable configuration (C_{A}) and being bent in a second direction away from the body (101) of the micro-robot (100) in the second stable configuration (C_{B}).

7. Micro-robot (100) according to any one of the preceding claims, **wherein** the steering structure (18) is a retractable steering foil (18) displaying an open and a retracted configuration with regards to the body (101) of the micro-robot (100), the steering foil (18) configuration being determined by the steering state (A, A₁, A₂, B) of the resonating structure (16):
- the non-activated steering state (B) of the resonating structure (16) induces the retracted configuration of the retractable steering foil (18), and
- the activated steering state (A, A₁, A₂) of the resonating structure (16) induces the open configuration of the retractable steering foil (18).

8. Micro-robot (100) according to claim **5, wherein** each multi-stable element (22) is a multi-stable shell (22) comprising a stack of several sheets.

9. Micro-robot (100) according to anyone of the preceding claims, **wherein** the steering system (10) further comprises at least one mobile cilium (24), the at least one cilium (24) being configured to be put in motion by the vibration of the body (101) of the micro-robot (100).

10. Micro-robot (100) according to the precedent claim, **wherein** the resonating structure (16) comprises at least one strand of a propulsion spring (14) comprised within the body (101).

11. Micro-robot (100) according to the precedent claim, **wherein** the activation of the resonating structure (16) in its at least one activated steering state (A, A₁, A₂) induces the retractation of the at least one strand of the propulsion spring (14).

12. Micro-robot (100) according to claims **10** or **11, wherein** the body (101) of the micro-robot (10) presents a global circular symmetry along a propulsion axis parallel to the propulsion direction, wherein the resonating structure (16) is further part of said body (101), and wherein the activation of the resonating structure (16) into one of its activated steering states (A, A₁, A₂) induces a break in the global circular symmetry of the body (101).

13. Micro-robot (100) according to claim **9, wherein** the at least one cilium (24) is part of the resonating structure (16), the at least one cilium (24) presenting a first movement intensity (I_{B}) in the non-activated steering state (B) and a second movement intensity (I_{A}) in the activated steering state (A, A₁, A₂), the second movement intensity (I_{A}) being different than the first movement intensity (I_{B}).

14. Micro-robot (100) according to the preceding claim, **wherein** each cilium (24) comprises a weight-resonator (20).

## Patentansprüche

1. Mikroroboter (100), der so konfiguriert ist, dass er sich entlang einer Antriebsrichtung durch Vibrationen bewegt, wobei der Mikroroboter (100) einen Körper (101) umfasst, der einen Aktuator (12) umfasst, der so konfiguriert ist, dass er Vibrationen erzeugt, die eine Bewegung des Mikroroboters (100) bewirken,
wobei der Mikroroboter (100) ein Lenksystem (10) umfasst, das eine Resonanzstruktur (16) umfasst, die so konfiguriert ist, dass sie an dem Mikroroboter (100) befestigt werden kann, wobei die Resonanzstruktur (16) umfasst:
- eine Lenkstruktur (18), die dazu dient, die Vortriebsrichtung zu steuern,
- eine Verteilung von Gewichtsresonatoren (20), wobei jeder Gewichtsresonator (20) so konfiguriert ist, dass er durch eine geeignete Aktivierungsresonanzfrequenz (f_{A}, f_{A1}, f_{A2}) aktiviert wird, wobei die jeweiligen geeigneten Aktivierungsresonanzfrequenzen (f_{A}, f_{A1}, f_{A2}) der Gewichtsresonatoren (20) voneinander verschieden sind,
**wobei** der Aktuator (12) so konfiguriert ist, dass er Schwingungen in einem Frequenzbereich erzeugt, der die geeignete Aktivierungsresonanzfrequenz jedes Gewichtsresonators umfasst,
**wobei** die Resonanzstruktur (16) mindestens zwei Zustände aufweist:
- mindestens einen aktivierten Lenkzustand (A, A₁, A₂), in dem mindestens einer der Gewichtsresonatoren mit der richtigen Aktivierungsresonanzfrequenz aktiviert wird, so dass die Antriebsrichtung des Mikroroboters (100) geändert wird,
- einen nicht aktivierten Lenkzustand (B), in dem keiner der Gewichtsresonatoren mit seiner eigenen Aktivierungsresonanzfrequenz aktiviert wird,so dass die Antriebsrichtung des Mikroroboters (100) beizubehalten wird.

2. Mikroroboter (100) nach dem vorhergehenden Anspruch, **wobei** der Aktuator (12) ein Vibrationsmotor (12) ist, der eine Spule (120) umfasst, die sich entlang der Antriebsrichtung (X) erstreckt und einen Magneten (121) umgibt, wobei das Magnet (121) durch die Spule (120) aktiviert werden kann und so konfiguriert ist, dass es sich entlang der Antriebsrichtung (X) hin und her bewegt.

3. Mikroroboter (100) nach einem der vorhergehenden Ansprüche, **wobei** die Resonanzstruktur (16) mehrere aktivierte Lenkzustände (A₁, A₂) aufweist, wobei jedem aktivierten Lenkzustand (A₁, A₂) eine andere geeignete Aktivierungsresonanzfrequenz (f_{A1}, f_{A2}) zugeordnet ist.

4. Mikroroboter (100) nach einem der vorhergehenden Ansprüche, **wobei:**
- der mindestens eine aktivierte Lenkzustand (A, A₁, A₂) ein Zustand ist, in dem die Konfiguration und die Bewegung der Resonanzstruktur (16) darauf abzielen, die Vortriebsrichtung des Mikroroboters (100) zu ändern,
- der nicht aktivierte Lenkzustand (B) ein Zustand ist, in dem die Konfiguration und die Bewegung der Resonanzstruktur (16) darauf abzielen, die Antriebsrichtung des Mikroroboters (100) beizubehalten.

5. Mikroroboter (100) nach einem der vorhergehenden Ansprüche, wobei die Resonanzstruktur (16) ferner eine Verteilung von multistabilen Elementen (22) umfasst, wobei jedes multistabile Element (22) zwischen mindestens zwei stabilen Konfigurationen verformbar ist:
- mindestens einer ersten stabilen Konfiguration (C_{A}), wenn sich die Resonanzstruktur (16) in ihrem aktivierten Lenkzustand (A) befindet,
- mindestens einer zweiten stabilen Konfiguration (C_{B}), wenn sich die Resonanzstruktur (16) in ihrem nicht aktivierten Lenkzustand (B) befindet.

6. Mikroroboter (100) nach dem vorhergehenden Anspruch, wobei jedes multistabile Element (22) ein bi-stabiler vorkomprimierter Balken (22) ist, der die erste und die zweite stabile Konfiguration (C_{A}, C_{B}) aufweist, wobei der vorkomprimierte Balken (22) in der ersten stabilen Konfiguration (C_{A}) in eine erste Richtung zum Körper (101) des Mikroroboters (100) hin gebogen ist und in der zweiten stabilen Konfiguration (C_{B}) in eine zweite Richtung vom Körper (101) des Mikroroboters (100) weg gebogen ist.

7. Mikroroboter (100) nach einem der vorhergehenden Ansprüche, wobei die Lenkstruktur (18) eine einziehbare Lenkfolie (18) ist, die eine offene und eine eingezogene Konfiguration in Bezug auf den Körper (101) des Mikroroboters (100) aufweist, wobei die Konfiguration der Lenkfolie (18) durch den Lenkzustand (A, A1, A2, B) der Resonanzstruktur (16) bestimmt wird:
- der nicht aktivierte Lenkzustand (B) der Resonanzstruktur (16) die eingezogene Konfiguration der einziehbaren Lenkfolie (18) bewirkt, und
- der aktivierte Lenkzustand (A, A1, A2) der Resonanzstruktur (16) die offene Konfiguration der einziehbaren Lenkfolie (18) hervorruft.

8. Mikroroboter (100) nach Anspruch **5, wobei** jedes multistabile Element (22) eine multistabile Schale (22) ist, die einen Stapel aus mehreren Blättern umfasst.

9. Mikroroboter (100) nach einem der vorhergehenden Ansprüche, **wobei** das Lenksystem (10) mindestens ein bewegliches Zilium (24) umfasst, wobei das mindestens eine Zilium (24) so konfiguriert ist, dass es durch die Vibration des Körpers (101) des Mikroroboters (100) in Bewegung gesetzt wird.

10. Mikroroboter (100) nach dem vorhergehenden Anspruch, **wobei** die Resonanzstruktur (16) mindestens einen Strang einer Antriebsfeder (14) umfasst, die in dem Körper (101) enthalten ist.

11. Mikroroboter (100) nach dem vorhergehenden Anspruch, **wobei** die Aktivierung der Resonanzstruktur (16) in ihrem mindestens einen aktivierten Lenkzustand (A, A₁, A₂) das Einziehen des mindestens einen Strangs der Antriebsfeder (14) induziert.

12. Mikroroboter (100) nach Anspruch **10** oder **11, wobei** der Körper (101) des Mikroroboters (10) eine globale kreisförmige Symmetrie entlang einer Antriebsachse parallel zur Antriebsrichtung aufweist, wobei die Resonanzstruktur (16) ein weiterer Teil des Körpers (101) ist, und wobei die Aktivierung der Resonanzstruktur (16) in einen ihrer aktivierten Steuerzustände (A, A₁, A₂) einen Bruch in der globalen kreisförmigen Symmetrie des Körpers (101) induziert.

13. Mikroroboter (100) nach Anspruch **9,** wobei das mindestens eine Zilium (24) Teil der Resonanzstruktur (16) ist, wobei das mindestens eine Zilium (24) eine erste Bewegungsintensität (I_{B}) in dem nicht aktivierten Lenkzustand (B) und eine zweite Bewegungsintensität (I_{A}) in dem aktivierten Lenkzustand (A, A₁, A₂) aufweist, wobei die zweite Bewegungsintensität (I_{A}) sich von der ersten Bewegungsintensität (I_{B}) unterscheidet.

14. Mikroroboter (100) nach dem vorhergehenden Anspruch, **wobei** jedes Zilium (24) einen Gewichtsresonator (20) umfasst.

## Revendications

1. Micro-robot (100) configuré pour se déplacer le long d'une direction de propulsion (X) par vibrations, ledit micro-robot (100) comprenant un corps (101) comprenant un actionneur (12) configuré pour générer des vibrations provoquant le déplacement du micro-robot (100),
ledit micro-robot (100) comprenant en outre un système de guidage (10) qui comprend une structure résonante (16) configurée pour être fixée au micro-robot (100), la structure résonante (16) comprenant :
- une structure de guidage (18) destinée à contrôler la direction de propulsion,
- une distribution de poids-résonateurs (20), chaque poids-résonateur (20) étant configuré pour être activé par une fréquence de résonance d'activation propre (f_{A}, f_{A1}, f_{A2}), les fréquences de résonance d'activation propres respectives (f_{A}, f_{A1}, f_{A2}) des poids-résonateurs (20) étant différentes les unes des autres,
**caractérisé en ce que** l'actionneur (12) est configuré pour générer des vibrations dans une gamme de fréquences comprenant la fréquence de résonance d'activation propre de chaque poids-résonateur,
**caractérisé en ce que** la structure résonante (16) présente au moins deux états :
- au moins un état activé de guidage (A, A₁, A₂), dans lequel au moins un des poids-résonateurs est activé à la fréquence de résonance d'activation propre de manière à changer la direction de propulsion du micro-robot (100),
- un état non-activé de guidage (B), dans lequel aucun des résonateurs de poids n'est activé à sa fréquence de résonance d'activation propre de manière à maintenir la direction de propulsion du micro-robot (100).

2. Micro-robot (100) selon la revendication précédente, dans lequel l'actionneur (12) est un moteur vibrant (12) qui comprend une bobine (120) s'étendant le long de la direction de propulsion (X) et entourant un aimant (121), l'aimant (121) étant activable par la bobine (120) et étant configuré pour se déplacer d'avant en arrière le long de la direction de propulsion (X).

3. Micro-robot (100) selon l'une quelconque des revendications précédentes, dans lequel la structure résonante (16) présente plusieurs états activés de guidage (A₁, A₂), chaque état activé de guidage (A₁, A₂) étant associé à une fréquence de résonance d'activation propre différente (f_{A1}, f_{A2}).

4. Micro-robot (100) selon l'une quelconque des revendications précédentes, dans lequel :
- l'au moins un état activé (A, A₁, A₂), est un état dans lequel la configuration et le mouvement de la structure résonante (16) visent à changer la direction de propulsion du micro-robot (100),
- l'état non-activé de guidage (B) est un état dans lequel la configuration et le mouvement de la structure résonante (16) visent à maintenir la direction de propulsion du micro-robot (100).

5. Micro-robot (100) selon l'une quelconque des revendications précédentes, dans lequel la structure résonante (16) comprend en outre une distribution d'éléments multi-stables (22), chaque élément multi-stable (22) étant déformable entre au moins deux configurations stables :
- au moins une première configuration stable (C_{A}) lorsque la structure résonante (16) est dans son état de direction activée (A),
- au moins une deuxième configuration stable (C_{B}) lorsque la structure résonante (16) est dans son état de direction non activée (B).

6. Micro-robot (100) selon la revendication précédente, dans lequel chaque élément multi-stable (22) est une poutre pré-comprimée bi-stable (22) présentant la première et la deuxième configurations stables (C_{A}, C_{B}), la poutre pré-comprimée (22) étant pliée dans une première direction vers le corps (101) du micro-robot (100) dans la première configuration stable (C_{A}) et pliée dans une deuxième direction écartée du corps (101) du micro-robot (100) dans la deuxième configuration stable (C_{B}).

7. Micro-robot (100) selon l'une quelconque des revendications précédentes, dans lequel la structure de guidage (18) est une feuille de guidage rétractable (18) présentant une configuration ouverte et une configuration rétractée par rapport au corps (101) du micro-robot (100), la configuration de la feuille de guidage (18) étant déterminée par l'état de guidage (A, A₁, A₂, B) de la structure résonante (16) :
- l'état non-activé de guidage (B) de la structure résonante (16) induit la configuration rétractée de la feuille de guidage rétractable (18), et
- l'état activé de guidage (A, A₁, A₂) de la structure résonante (16) induit la configuration ouverte de la feuille de guidage rétractable (18).

8. Micro-robot (100) selon la revendication 5, dans lequel chaque élément multi-stable (22) est une coque multi-stable (22) comprenant un empilement de plusieurs feuilles.

9. Micro-robot (100) selon l'une quelconque des revendications précédentes, dans lequel le système de pilotage (10) comprend en outre au moins un cil mobile (24), l'au moins un cil (24) étant configuré pour être mis en mouvement par la vibration du corps (101) du micro-robot (100).

10. Micro-robot (100) selon la revendication précédente, dans lequel la structure résonante (16) comprend au moins un brin d'un ressort de propulsion (14) compris dans le corps (101).

11. Micro-robot (100) selon la revendication précédente, dans lequel l'activation de la structure résonante (16) dans son au moins un état activé de guidage (A, A₁, A₂) induit la rétractation de l'au moins un brin du ressort de propulsion (14).

12. Micro-robot (100) selon les revendications **10 ou 11,** dans lequel le corps (101) du micro-robot (10) présente une symétrie circulaire globale le long d'un axe de propulsion parallèle à la direction de propulsion, dans lequel la structure résonante (16) fait également partie dudit corps (101), et dans lequel l'activation de la structure résonante (16) dans l'un de ses états activés de guidage (A, A₁, A₂) induit une rupture de la symétrie circulaire globale du corps (101).

13. Micro-robot (100) selon la revendication **9,** dans lequel l'au moins un cil (24) fait partie de la structure résonante (16), l'au moins un cil (24) présentant une première intensité de mouvement (I_{B}) dans l'état non-activé de guidage (B) et une deuxième intensité de mouvement (I_{A}) dans l'état activé de guidage (A, A₁, A₂), la deuxième intensité de mouvement (I_{A}) étant différente de la première intensité de mouvement (I_{B}).

14. Micro-robot (100) selon la revendication précédente, dans lequel chaque cil (24) comprend un poids-résonateur (20).
